# EUROPEAN PATENT APPLICATION

(11) **EP 2 260 808 A1**
(43) Date of publication of application: **15.12.2010**
(21) Application number: 09715883.6
(22) Date of filing: 27.01.2009
(51) Int. Cl.: A61F 13/15, A61F 13/472, A61F 13/53, A61F 13/534, A61F 13/539

(54) **ABSORPTIVE ARTICLE**

(30) Priority: 25.02.2008 JP 2008043588
(71) Applicant: Unicharm Corporation, Ehime 799-0111 (JP)
(72) Inventor: The designation of the inventor has not yet been filed
(74) Representative: Peter, Julian
(86) International application number: PCT/JP2009/051240
(87) International publication number: WO 2009/107435

(57) **Abstract**

An absorbent article has a liquid permeable top sheet (5) making contact with the wearer's skin surface, a liquid impermeable back sheet (6) making contact with the surface of underwear, and an absorber (7) mounted between the top sheet (5) and the back sheet (6) and capable of absorbing a bodily liquid. The an absorber (7) has a projected portion (2) which is a portion formed on a lower layer absorber (8) by overlaying an upper layer absorber (9) on the lower layer absorber (8), and the upper layer absorber (9) has smaller dimensions in the longitudinal and lateral directions than the lower layer absorber (8). A first recessed portion (3) is provided around the projected portion (2) so as to surround the projected portion (2). The first recessed portion (3) is formed by a continued portion (3a) and an intermittent portion (4).

## Description

### [Technical Field]

The present invention relates to an absorbent article used for a disposable paper diaper, a sanitary napkin, a panty-liner, an incontinent pad or the like.

### [Background Art]

Patent Literature 1 discloses a sanitary napkin as a conventional absorbent article. This absorbent article has a projected portion and a recessed portion. The projected portion is in a solid shape and is formed on a skin contact surface, and the recessed portion is formed of a groove on a surrounding surface outside the projected portion. Accordingly, the sanitary napkin has a structure in which the recessed portion is provided to surround the entire circumference of the projected portion. In such a structure, since the recessed portion is formed to surround the entire circumference of the projected portion, menstrual blood flowing from the projected portion can be trapped by the recessed portion, so that the menstrual blood can be prevented from leaking sideward, frontward and rearward from the entire circumference of the absorbent article.
Patent Literature 1: JP Patent No. 3868628

### [Disclosure of the Invention]

However, a conventional absorbent article has a recessed portion formed of a top sheet and an adsorbent layer being compressed, and thus the density of the recessed portion is higher than that of a circumference thereof. Accordingly, the rigidity of the recessed portion is higher than that of the circumference thereof. When the rigidity of the recessed portion is high, the absorbent material is not easily bent in a direction crossing to a direction in which the recessed portion continues. Thus, the absorbent article needs to be forcibly folded because it is not possible to define a portion of the absorbent article to be bent in accordance with the curve of a body. As a result, this causes problems of twists of the absorbent article and an unpleasant texture.

In this regard, an object of the present invention is to provide an absorbent article that allows a folded portion to be defined even in a structure in which a recessed portion is formed by surrounding a projected portion, thereby preventing an occurrence of a twist and providing a pleasant texture.

### [Summary of Invention]

An aspect of the invention is summarized as an absorbent article, including: a top sheet being liquid permeable and adopted to contact a skin surface; a back sheet being liquid impermeable and adopted to contact an undergarment surface; and an absorber provided between the top sheet and the back sheet, being able to absorb a body fluid, and including a projected portion formed of an upper layer absorber and a lower layer absorber, while the upper layer absorber being smaller than the lower layer absorber in a longitudinal direction and a width direction of the absorbent article is laminated on the lower layer absorber, and a first recessed portion provided in a circumference of the projected portion so as to surround the projected portion, wherein the first recessed portion includes a continued portion in which the first recessed portion is continued and an intermittent portion in which the first recessed portion is discontinued.

In the aspect of the invention, the first recessed portion may be formed in a substantially symmetric position with respect to a center axis in the longitudinal direction, and the first recessed portion may be continued on one side with respect to the center axis, and the first recessed portion includes the intermittent portion on an other side with respect to the center axis.

In the aspect of the invention, the first recessed portion may be formed in a position substantially symmetric with respect to a center axis in the longitudinal direction, and the intermittent portion may be respectively formed at both sides of the center axis.

In the aspect of the invention, the absorbent article may further includes a second recessed portion provided in a region outside the first recessed portion, and the second recessed portion may be formed continuously or discontinuously from a vicinity of the intermittent portion to an opposite side of the intermittent portion, with respect to the center axis in the width direction.

In the aspect of the invention, the second recessed portion may includes a deep portion and a deepest portions, a depth of the deepest portion may be different from a depth of the deep portion, and the deepest portion may be separately formed in a plurality of places.

In the aspect of the invention, the second recessed portion may be arranged toward an edge in the longitudinal direction.

In the aspect of the invention, the second recessed portion may be a groove provided on an edge side in the longitudinal direction, and the groove is arranged to extend from an intermittent portion side to an opposite side of the intermittent portion in the width direction, with respect to the center axis in the longitudinal direction.

In the aspect of the invention, the absorbent article may further include a flat portion provided in an area surrounded by the first recessed portion and adjacent to the projected portion, and a height of the flat portion may be lower than a height of the projected portion.

According to the absorbent article of the present invention, a discontinued intermittent portion is formed in a first recessed portion surrounding the projected portion. According to the absorbent article of the present invention, the intermittent portion defines a folded portion, thereby an occurrence of a twist can be prevented and the texture of the absorbent article can be improved.

### [Brief Description of Drawings]

Fig. 1 is a plan view showing an absorbent article of a first embodiment of the present invention.
Fig. 2 is a cross-sectional view taken along the A-A line in Fig. 1.
Fig. 3 is a cross-sectional view of a product.
Fig. 4 is a plan view showing an absorbent article of a second embodiment.
Fig. 5 is a plan view showing another absorbent article of the second embodiment.
Fig. 6 is a plan view showing an absorbent article of a third embodiment.
Fig. 7 is an enlarged plan view of a second recessed portion.
Fig. 8 is a plan view showing an absorbent article of a fourth embodiment.
Fig. 9 is a plan view showing an absorbent article of a fifth embodiment.

### [Description of Embodiments]

The present invention will be described in detail below by referring to an embodiment shown in the figures. Note that, in each embodiment, same reference numerals are given and corresponded to same members.

### (First Embodiment)

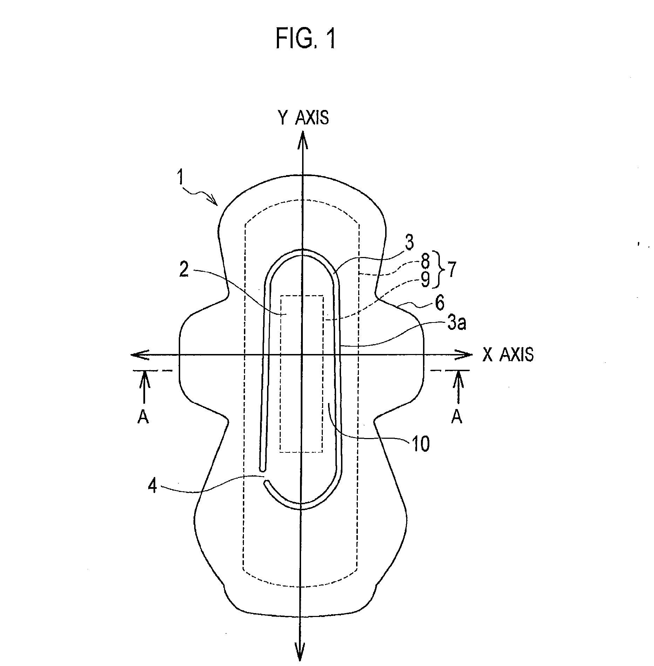

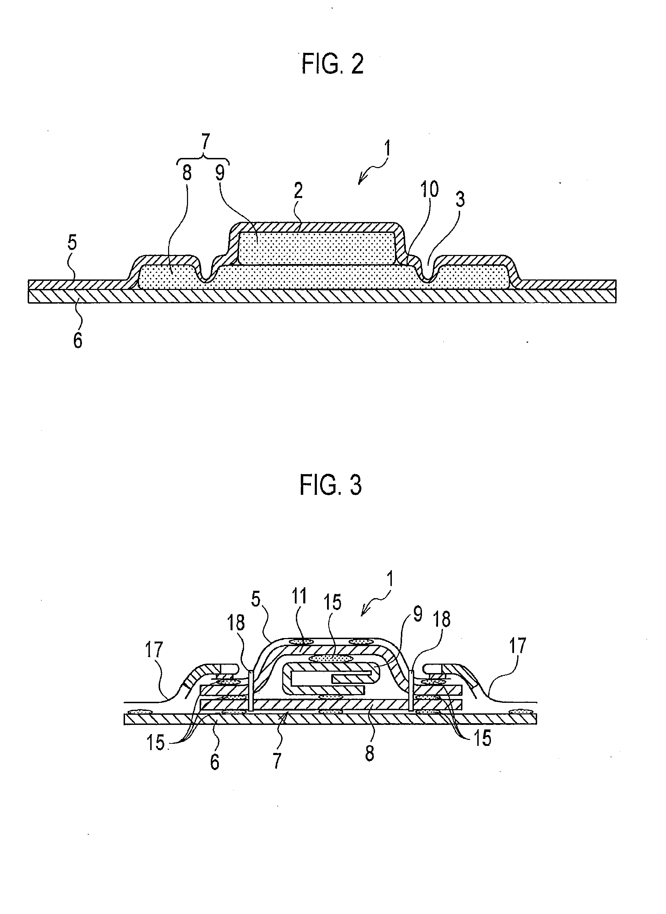

Figs. 1 to 3 shows a first embodiment of the present invention. Fig. 1 is a plan view. Fig. 2 is a cross-sectional view taken along the A-A line in Fig. 1. Fig. 3 is a cross-sectional view of a product. An absorbent article 1 can be used for, for example, sanitary napkins.

The absorbent article 1 includes a liquid permeable top sheet 5, a liquid impermeable back sheet 6, and an absorber 7 interposed between the top sheet 5 and the back sheet 6. When a wearer puts on the absorbent article 1, the top sheet 5 comes in contact with a skin surface of the wearer, and the back sheet 6 comes in contact with an undergarment of the wearer.

In the absorbent article 1 of this embodiment, as shown in Fig. 1, the X axis and the Y axis intersecting with each other are set. The Y axis is the center axis in the longitudinal direction. Both ends of the Y axis are respectively putted on a ventral side and hip side of a wearer. At this time, the X axis is in the right-left direction seen from the wearer.

A material that can be used for the top sheet 5 includes: a thermoplastic resin, such as polyethylene, polypropylene, or polyethylene terephthalate. These fibers can be used as a single resin or a complex synthetic fiber of core-in-sheath type, decentered type of the core-in-sheath, or side-by-side type. In addition, as another form of the top sheet, a perforated plastic sheet may be used. As the perforated plastic sheet, a perforated sheet made of a thermoplastic resin, such as PE, PP, or PET, a porous foamed material, or the like may be used. In addition, as the perforated plastic sheet, a whitish perforated plastic sheet in which an inorganic filler formed of titanium oxide or calcium carbonate is mixed in a range of 0.5 to 10 percent by mass, may be used when needed. Also, a perforated film in which a film formed of a thermoplastic resin is processed to be apertured by a perforation, a heat embossing process, a mechanical process or the like, may be used. This perforated film may be used as a complex sheet by being bonded with a nonwoven. In the top sheet 5, in order to control wettability, hydrophilic or a water-repellent solution can be adhered on or mixed in these fiber surfaces. Such an oil solution generally exerts its effect with its amount 0.2 to 1.0 weight%. These fibers are subjected to, for example, thermal sealing, water confounding, or spunbonding to form a single or complex nonwoven, and such nonwoven fabric may be used as the top sheet 5. When hydrophilicity with body fluid is considered, a cellulose-based liquid hydrophilic fiber, such as pulp, chemical pulp, rayon, acetate, or natural cotton, may be included. It is preferable that a fiber to be used is 1.1 to 6.6 dtex and the basis weight is adjusted in a range of 15 to 120 g/m². Note that, a sheet to be a cushion sheet or a diffusion sheet may be arranged between the top sheet 5 and the absorber 7.

A material that can be user for the back sheet 6 includes: polyethylene, polypropylene, polyethylene terephthalate, polyvinyl alcohol, polylactic acid, polybutyl succinate, or a nonwoven, paper, or a stacked material of these materials, with a thickness of 15 to 60 µm. In addition, the back sheet 6 may be a permeable film which can be obtained by performing a stretching process with the inorganic filler being filled therein. Specifically, a film which is mainly formed of a polyethylene resin with a low density and is adjusted in a basis weight in a range of 15 to 30 g/m² may be selected.

The absorber 7 includes a lower layer absorber 8, an upper layer absorber 9 laminated on the lower absorber 8, and an uppermost layer absorber 11 laminated on the upper layer absorber 9. The upper layer absorber 9 is smaller than the lower layer absorber 8 in a longitudinal direction and a width direction. The upper layer absorber 9 serves as a projected portion 2 projecting on the lower layer absorber 8. The uppermost layer absorber 11 is laminated on the upper layer side of the projected portion 2, which is the lower side of the top sheet 5. Note that, the lower absorber 8 and the upper absorber 9 may be integrally formed instead of being separated. In addition, the lower layer absorber 8, the upper layer absorber 9, and the uppermost absorber 11 are bonded with one another with a hot melt 15.

The absorber 7 formed of the lower layer absorber 8, the upper layer absorber 9, and the uppermost layer absorber 11 is made of a material that can absorb a body fluid such as menstrual blood. As the absorber 7, material such as pulp, chemical pulp, rayon, acetate, natural cotton, polymer absorber, fibrous polymer absorber, synthetic fiber, or cellular porous medium, is used as a signal material or a mixed material of these materials, so that the absorber 7 can be hardly deformed and have less chemical irritant. In addition, a material in which a molecular orientation is increased by being stretched at the time of spinning or a material having a different type with its cross section in a Y shape or in a C shape may be mixed into these materials. Furthermore, an oil solution may be applied to or contained in the fiber so as to increase smoothness among the fibers. In the absorber 7, the above-described material may be formed in a sheet form by air laid or may be a sheet having at least one layer.

Note that, in Fig. 3, side sheets 17 are arranged on both sides of the top sheet 5, the uppermost layer absorber 11, and the lower layer absorber 8. Here, the side sheets 17 are bonded to the back sheet 6 with the hot melt 15, together with the top sheet 5, the uppermost absorber 11, and the both sides of the lower layer absorber 8.

In this embodiment, the absorber 7 is formed in such a manner that the above-described material is laminated, then the laminated body is rolled with a sheet material (covering material), or is sandwiched between the upper and lower sheets (covering materials), so that the laminated body is covered, and then pressed. As the material covering the laminated body, usable are nonwoven made of: a cellulose-based fiber such as pulp, chemical pulp, rayon, acetate, or natural cotton; a thermoplastic resin such as polyethylene, polypropylene, or polyethylene terephthalate as a single resin; or a fiber of core-in-sheath type, decentered type of core-in-sheath, or side-by-side type as a single fiber or a complex fiber. The nonwoven is formed by water confounding, spundonding, trough air, or the like.

The projected portion 2 can be formed by arranging the absorber formed by the above-described method on the absorber having at least one layer. Alternatively, the projected portion 2 may be arranged on or under the absorber having one layer or may be arranged between at least two layers of the absorber. Furthermore, the projected portion 2 may be formed within one-layer absorber by partially increasing a basis weight than that in the circumference.

The projected portion 2 preferably has the size equal to or larger than 40 mm in the Y axis direction, and more preferably 60 to 120 mm. The projected portion 2 preferably has the size equal to or larger than 10 mm in the X axis direction, and more preferably 25 to 50 mm. As to a positional relationship, the projected portion 2 on the absorber 7 is preferably located with its front end portion in the Y axis direction at least 20 mm posterior from the front end portion in the Y axis direction. The end portion of the projected portion 2 in the Y axis direction is preferably positioned in at least 30 mm posterior from the front end portion of the absorber 7 in the Y axis direction. Note that, the shape of the projected portion 2 is not particularly limited and may be any shape, such as rectangular, elliptic, circular, drop-shaped, square, or triangular.

A first recessed portion 3 formed of a groove is provided so as to surround the projected portion 2 (position shown by reference numeral 18 in Fig. 3). The first recessed portion 3 formed of a groove is provided in the lower layer absorber 8 and the uppermost layer absorber 11 so as to be positioned in the circumference of the projected portion 2 and is formed by compressing the top sheet 5, the lower layer absorber 8, and the uppermost layer absorber 11. Accordingly, the first recessed portion 3 is not formed in the upper layer absorber 9.

In this embodiment, a flat portion 10, which is lower than the projected portion 2, is formed in the region surrounded by the first recessed portion 3. By forming the flat portion 10, body fluid from the projected portion 2 can be smoothly led to the first recessed portion 3.

The first recessed portion 3 is formed in a substantially symmetric position with respect to the Y axis, which is the center axis in the longitudinal direction. On one side (right side) of the Y axis, a continued portion 3a is formed so that the recessed portion 3 is continued, but on the other side (left side), an intermittent portion 4 is provided so that the recessed portion 3 is discontinued. In other words, the first recessed portion 3 does not surround the entire circumference of the projected portion 2. The intermittent portion 4 is not provided along the X axis direction but is provided along the Y axis direction. Since the movement of the wearer is large in the vicinity of the leg opening portion, the wearer will feel uncomfortable when putting on the absorbent article 1. Accordingly, the wearer will feel less likely uncomfortable if a foldable region is provided near the leg opening portion.

The size of the intermittent portion 4 is in a range of 2 to 20 mm, and preferably 5 to 10 mm. If the size is less than 2 mm, the absorber 7 cannot be sufficiently folded. If the size is more than 20 mm, menstrual blood leaking from the projected portion 2 cannot be trapped and the menstrual blood easily leaks out to the outside of the first recessed portion 3.

In the absorbent article 1 formed in the above-described structure, the projected portion 2 absorbs menstrual blood. The menstrual blood that cannot be absorbed by the projected portion 2 is trapped by the first recessed portion 3 surrounding the projected portion 2. The first recessed portion 3 has a high rigidity, and prevents the absorbent article 1 from being folded in accordance with the body movement. However, in this embodiment, the first recessed portion 3 is provided with the intermittent portions 4 without surrounding the entire circumference of the projected portion 2. For this reason, the rigidity of the intermittent portion 4 is lower than that of the first recessed portion 3, so the absorbent article 1 is allowed to be easily folded by the intermittent portion 4 and to be soft and flexible. The intermittent portion 4 is provided only on one side of the center axis (Y axis) in the longitudinal direction. On the other side, the groove of the first recessed portion 3 is continued. Therefore, the intermittent portion 4 is folded preferentially, and any other portions can be folded at any positions in accordance with various body movements. Accordingly, an occurrence of the twist of the absorbent article 1 can be prevented, and the texture thereof can be improved.

In this embodiment, the intermittent portion 4 is provided in a posterior direction of the rear edge (dorsal side of the wearer when being put on) of the projected portion 2 in the Y axis direction. For this reason, even if menstrual blood that cannot be absorbed by the projected portion 2 flows to the right and left (in the X axis direction) of the projected portion 2, the groove of the first recessed portion 3 is positioned on the right and left (in the X axis direction) of the projected portion 2, so that the menstrual blood can be trapped. The menstrual blood flown from the projected portion 2 in the Y axis direction can be also trapped by the first recessed portion 3 on the Y axis.

### (Second Embodiment)

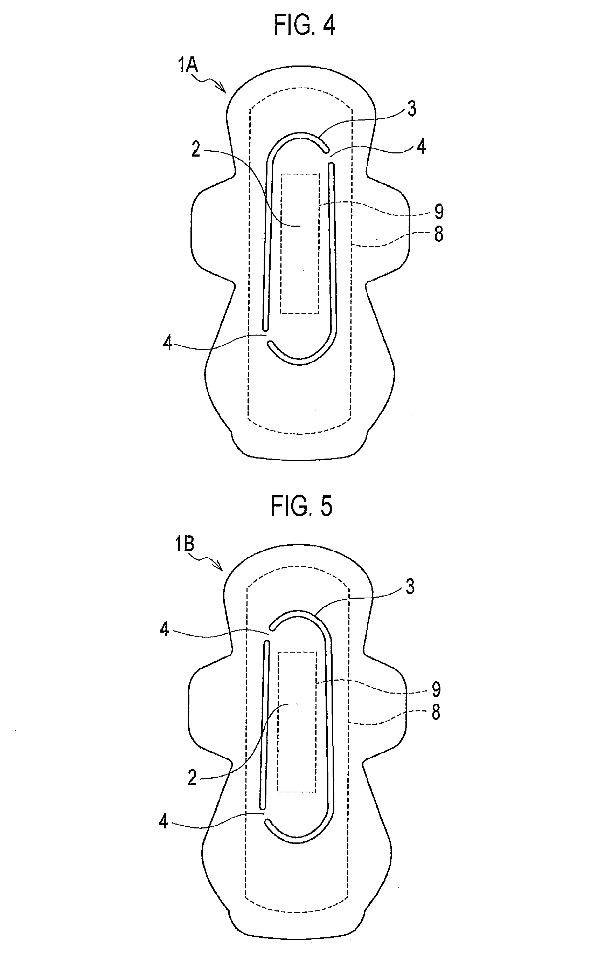

Fig. 4 and Fig. 5 show absorbent articles 1A and 1 B of a second embodiment of the present invention.

In these figures, two intermittent portions 4 are provided with respect to a first recessed portion 3. In the absorbent article 1A of Fig. 4, the two intermittent portions 4 (a rear intermittent portion 4 and a front intermittent portion 4) are provided respectively on both sides of the center axis (Y axis) in the longitudinal direction. In the absorbent article 1 B of Fig. 4, the intermittent portions 4 are provided on the same side.

In these figures, the front intermittent portion 4 is provided in an anterior region (ventral side of the wearer when being put on) than the front end portion of the projected portion 2 in the Y axis direction when the absorbent article 1 is putted on. In the X axis direction, the intermittent portion 4 is provided in a region different from the Y axis center line. It is preferable that the intermittent portion 4 is formed not on a groove provided in the X axis direction, but in one portion of the groove provided in the Y axis direction. The size of the intermittent portion 4 is similar to the intermittent portion 4 according to the first embodiment.
Accordingly, the intermittent portion 4 can follow the body movements in the front portion (ventral side of the wearer when being put on). Note that, for a purpose of trapping menstrual blood, providing the intermittent portions 4 on different sides respectively prevents menstrual blood from leaking from only one side, as in the absorbent material 1A of Fig. 4. It is also preferable to provide the intermittent portions 4 on different sides with regard to rigidity because the rigidity of only one of the right and left directions of the wearer is not particularly weakened.

### (Third Embodiment)

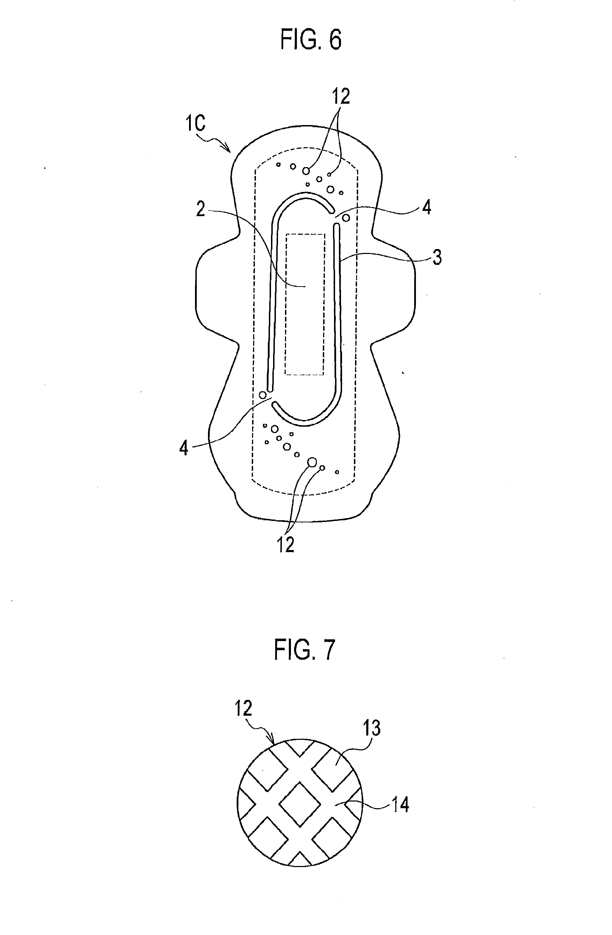

Fig. 6 shows an absorbent article 1C of a third embodiment of the present invention. In this embodiment, in addition to the shape of the second embodiment, a plurality of second recessed portions 12 is provided in front portion (a portion that comes to the ventral side with respect to the center of the absorbent article when being put on by the wearer) and a rear portion (a portion that comes to the dorsal side with respect to the center of the absorbent article when being put on by the wearer) of the absorbent article 1C. The plurality of second recessed portions 12 provided on the front portion is provided with intervals, on the side opposite to the intermittent portions 4 side with respect to the Y axis, while extending from vicinities of the intermittent portions 4 to a front edge side in the Y axis direction of the absorber 7. The plurality of second recessed portions 12 provided on the rear portion is provided with intervals, on the side opposite to the intermittent portions 4 side with respect to the Y axis, while extending from vicinities of the intermittent portions 4 to a rear edge side in the Y axis direction of the absorber 7.

The second recessed portion 12 is formed by compressing the top sheet 5 and the absorber 7. For this reason, the second recessed portion 12 has its density higher than that in the circumference. Further, the second recessed portion 12 has strength to absorb liquid. Therefore, even if menstrual blood leaks from the intermittent portions 4, the menstrual blood is led to the direction in which the second recessed portions 12 are provided. Accordingly, the entire surface of the absorber 7 can be effectively used, so that the menstrual blood is prevented from leaking outward from the absorbent article 1C. In addition, the recessed portions 12 are provided with being spaced apart. Thus, the second recessed portion 12 does not interrupt the body movement, and allows any portions to be folded in accordance with various body movements.

Among the second recessed portions 12, the closest to the intermittent portion 4 is provided in a distance of 2 to 30 mm from the intermittent portion 4. If the distance is less than 2 mm, the intermittent portion 4 cannot allow the absorbent article 1C to be folded. If the distance exceeds 30 mm away the intermittent portion 4, the menstrual blood cannot be sufficiently guided. For a similar reason, a distance between the individual portions of the second recessed portions 12 is also 2 to 30 mm. It is preferable that the size of each recessed portion 12 be in a range of 0.5 to 10 mm in a broader portion. If the size is in a range less than 0.5 mm, strength to absorb the menstrual blood is weak. If the size exceeds 10 mm, the texture is deteriorated and thus brings discomfort.

Fig. 7 shows the second recessed portion 12 in an enlarged form. The second recessed portion 12 may be a recessed portion having the same depth on the entire surface thereof. However, the second recessed portion 12 of this embodiment is formed by an assembly including a deepest portion 13 and a deep portion 14. Here, the depth of the deepest portion 13 is different from the deep portion 14. According to this second recessed portion 12, the density of the deepest portion 13 and the density of the deep portion 14 are different. Thus, it is effective to absorb the menstrual blood, and the rigidity does not become excessively high and a texture thereof is less likely to be deteriorated.

The shape of the second recessed portion 12 is not limited to the shape of Fig. 7, and may be in another shape. The second recessed portion 12 is provided so that at least a part thereof would be surely present on a virtual line, assuming that the virtual line is provided so as to be vertical to the Y axis. The distance between the edge of the absorber 7 and the second recessed portion 12 positioned in the closest to the edge is equal to or longer than 5 mm. If the distance is less than 5 mm, the menstrual blood is excessively guided to the edge of the absorber 7, and the guided blood may leak from the absorbent article 1C.

### (Fourth Embodiment)

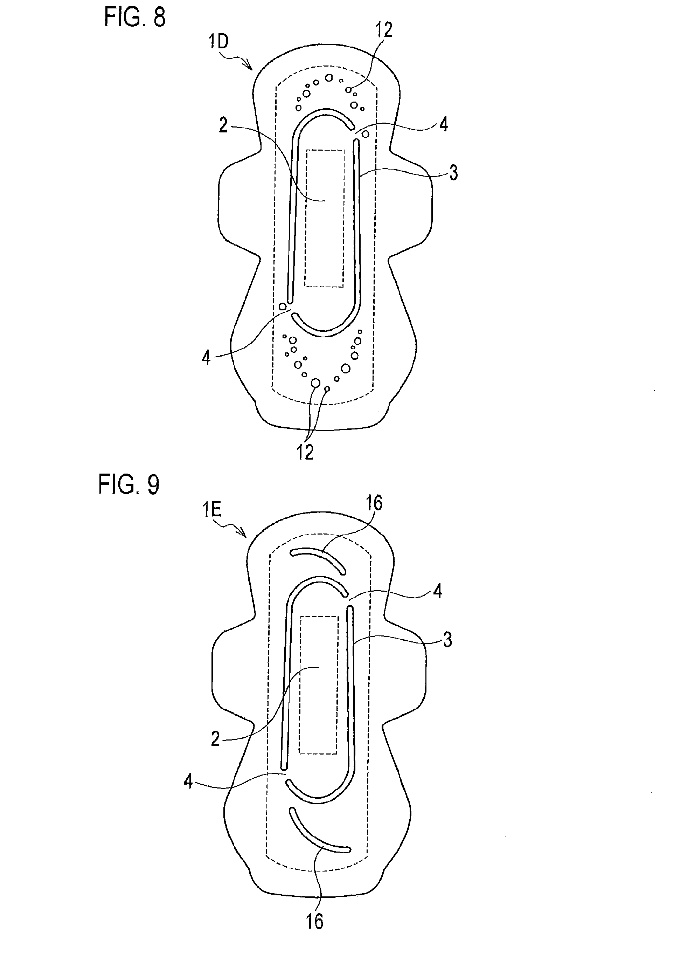

Fig. 8 shows a fourth embodiment of the present invention. An absorbent article 1 D of the present embodiment is a modification of the third embodiment. In the absorbent article 1D, second recessed portions 12 are discontinuously provided toward an edge of the center axis of the Y axis, unlike the absorbent article 1C of Fig. 6. Additionally, the second recessed portions 12 are discontinuously provided from the edge of the Y axis toward the center in the X axis direction on the different side in X axis direction from the side on which the second recessed portion 12 is provided toward the edge of the center axis of the X axis. Thus, similar to the absorbent article 1C of Fig. 6, menstrual blood is guided, and the entire surface of the absorber 7 can be effectively utilized.

In this case, it is preferable that the individual portions of the second recessed portions 12 be asymmetrically provided with respect to the Y axis. As a result, any portion is not preferentially folded and any portion can be folded. Accordingly, the absorbent article 1 D can be freely folded in accordance with body movements of the wearer.

### (Fifth Embodiment)

Fig. 9 shows an absorbent article 1 E of a fifth embodiment of the present invention. In the present embodiment, a second recessed portion 16 is formed by an arc-shaped continued groove. The second recessed portions 16 formed of the continued groove are provided in the front portion (the portion that comes to the ventral side with respect to the center of the absorbent article when being put on by the wearer) and the rear portion (the portion that comes to the dorsal side with respect to the center of the absorbent article when being put on by the wearer) of the absorbent article 1 E. In addition, the second recessed portion 16 is provided in a direction similar to that of the second recessed portion 12 of the absorbent article 1C of Fig. 6. For this reason, menstrual blood is guided along the second recessed portion 16, so that the entire surface of the absorber 7 can be effectively utilized.

The present invention is not limited to the present embodiments, and the first recessed portion 3 may have the one and the same depth or may have a deep portion and a deepest portion like the second projected and recessed portion.
In addition, the shape of the deepest portion may be a square, heart, star, or the like in addition to a circle.

This application is based upon the prior Japanese Patent Application No. JP 2008-043588, filed on February 25, 2008, the entire contents of which are incorporated herein by reference.

### [Industrial Applicability]

As mentioned above, according to the present invention, the intermittent portion defines a folded portion, thereby an occurrence of a twist can be prevented and the texture of the absorbent article can be improved, even if the recessed portion is provided surrounding the entire circumference of the projected portion 2.

## Claims

1. An absorbent article, comprising:
a top sheet being liquid permeable and adopted to contact a skin surface;
a back sheet being liquid impermeable and adopted to contact an undergarment surface; and
an absorber provided between the top sheet and the back sheet, being able to absorb a body fluid, and including a projected portion formed of an upper layer absorber and a lower layer absorber, while the upper layer absorber being smaller than the lower layer absorber in a longitudinal direction and a width direction of the absorbent article is laminated on the lower layer absorber, and
a first recessed portion provided in a circumference of the projected portion so as to surround the projected portion, wherein
the first recessed portion includes a continued portion in which the first recessed portion is continued and an intermittent portion in which the first recessed portion is discontinued.

2. The absorbent article according to claim 1, wherein
the first recessed portion is formed in a substantially symmetric position with respect to a center axis in the longitudinal direction,
the first recessed portion is continued on one side with respect to the center axis, and the first recessed portion includes the intermittent portion on an other side with respect to the center axis.

3. The absorbent article according to claim 1, wherein
the first recessed portion is formed in a position substantially symmetric with respect to a center axis in the longitudinal direction, and
the intermittent portion is respectively formed at both sides of the center axis.

4. The absorbent article according to claim 3, further comprising,
a second recessed portion provided in a region outside the first recessed portion, wherein
the second recessed portion is formed continuously or discontinuously from a vicinity of the intermittent portion to an opposite side of the intermittent portion with respect to the center axis in the width direction.

5. The absorbent article according to claim 4, wherein
the second recessed portion includes a deep portion and a deepest portion,
a depth of the deepest portion is different from a depth of the deep portion, and
the deepest portion is separately formed in a plurality of places.

6. The absorbent article according to claim 5, wherein
the second recessed portion is arranged toward an edge in the longitudinal direction.

7. The absorbent article according to claim 4, wherein
the second recessed portion is a groove provided on an edge side in the longitudinal direction, and the groove is arranged to extend from an intermittent portion side to an opposite side of the intermittent portion in the width direction, with respect to the center axis in the longitudinal direction.

8. The absorbent article according to claim 1, further comprising:
a flat portion provided in an area surrounded by the first recessed portion and adjacent to the projected portion, wherein
a height of the flat portion is lower than a height of the projected portion.
